# EUROPÄISCHE PATENTSCHRIFT

(11) **EP 3 462 164 B1**
(45) Veröffentlichungstag und Bekanntmachung des Hinweises auf die Patenterteilung: **20.03.2024**
(21) Anmeldenummer: 18188506.2
(22) Anmeldetag: 10.08.2018
(51) Int. Cl.: G01N 21/88, G01N 21/898, G01B 11/04, G01N 33/46

(54) **ANORDNUNG UND VERFAHREN ZUR INSPEKTION VON BEWEGTEN PLATTENFÖRMIGEN OBJEKTEN**
ASSEMBLY AND METHOD OF INSPECTING MOVED PLATE-SHAPED OBJECTS
DISPOSITIF ET PROCÉDÉ D'INSPECTION D'OBJETS EN FORME DE PLAQUE MOBILES

(30) Priorität: 29.09.2017 DE 102017009153
(43) Veröffentlichungstag der Anmeldung: 03.04.2019
(73) Patentinhaber: Baumer Inspection GmbH, 78467 Konstanz (DE)
(72) Erfinder: Detinkin, Igor, 78464 Konstanz (DE)
(74) Vertreter: Strauss, Steffen

(56) Entgegenhaltungen:
- EP-A1- 0 568 460
- EP-A1- 1 950 561
- EP-A1- 2 687 837
- EP-A2- 0 330 495
- EP-A2- 2 679 951
- WO-A1-00/62011
- US-A1- 2009 002 694

## Beschreibung

Die Erfindung betrifft eine Anordnung und ein Verfahren zur Inspektion von bewegten plattenförmigen Objekten.

Die plattenförmigen Objekte können beispielsweise Platten für Möbel sein. Solche plattenförmigen Objekte werden während der Produktion bearbeitet. Anschließend kann eine bearbeitete Fläche auf Defekte inspiziert werden. Problematisch ist allerdings, dass bei der Bearbeitung an Stellen oder Flächen, die in einem anderem Bearbeitungsschritt vorher bearbeitet wurden oder unbearbeitet blieben, oder ggf. bereits inspiziert worden sind durch eine weitere Bearbeitung Defekte auftreten können. Beispielsweise können diese Defekte an Flächen entstehen, die an bereits bearbeitete Flächen angrenzen. In der EP 0 330 495 A2 ist ein Inspektionssystem für Pakete, insbesondere zur Erfassung und Bewertung der Qualität von Packungen gezeigt. Mit dem Inspektionssystem werden hierbei auf einem Förderer transportierte identische Packungen mittels einer Lichtquelle beleuchtet und von mindestens einer Kamera erfasst, wobei die Kamera optisch senkrecht zu einer Oberfläche der jeweiligen Packung ausgerichtet ist und eine Analyseeinrichtung vorgesehen ist, die auf die Kameraausgangssignale anspricht, um die Kameraaufnahme hinsichtlich der Einhaltung der Packungsspezifikation auszuwerten.

Ferner ist aus der EP 2 679 951 A2 ein Inspektionssystem für Holzstücke bekannt, bei dem eine Spiegelanordnung verwendet wird, um mit einer Kamera mehrere Seiten abbilden zu können, während gemäß der WO 00 / 62011 A1 ein Inspektionssystem mit mehreren Kameras verwendet wird, um ein Bild aus mindestens drei Richtungen auf einmal aufzunehmen.

Aufgabe der Erfindung ist es, eine Lösung bereitzustellen, bei der auch eine Inspektion der plattenförmigen Objekte an Stellen oder Flächen, die in einem anderem Bearbeitungsschritt vorher bearbeitet wurden oder unbearbeitet blieben, oder ggf. bereits inspiziert worden sind, möglich ist.

Erfindungsgemäß wird dies gelöst, wie in Anspruch 1 definiert, durch eine Anordnung zur Inspektion von relativ zu einer Kameravorrichtung in einer Bewegungsrichtung durch wenigstens zwei Beobachtungsbereiche hindurch bewegten plattenförmigen Objekten, wobei die Anordnung die Kameravorrichtung umfasst, wobei in einem Erfassungszustand ein schräg entgegen der Bewegungsrichtung verlaufender Beobachtungsbereich auf einen, als Pixelstreifen ausgebildeten Abbildungsbereich der Kameravorrichtung abgebildet ist, wobei in einem weiteren Erfassungszustand ein weiterer Beobachtungsbereich auf einen weiteren Abbildungsbereich der Kameravorrichtung abgebildet ist, und wobei die Anordnung ferner einen Bildprozessor aufweist, der in den Erfassungszuständen aufgenommene streifenförmige Bilder zu zweidimensionalen Bildern zusammenfügt.

Ferner wird die Aufgabe gelöst, wie in Anspruch 7 definiert, durch ein Verfahren zur Inspektion von relativ zu einer Kameravorrichtung in einer Bewegungsrichtung durch Beobachtungsbereiche hindurch bewegten plattenförmigen Objekten, wobei in einem Erfassungszustand entlang eines, schräg entgegen der Bewegungsrichtung verlaufenden Beobachtungsbereichs streifenförmige Bilder aufgenommen werden und wobei in einem weiteren Erfassungszustand entlang eines weiteren Beobachtungsbereichs streifenförmige Bilder aufgenommen werden, und wobei jeweils die in den einzelnen Erfassungszuständen aufgenommenen streifenförmigen Bilder zu einem zweidimensionalen Bild zusammengesetzt werden.

Die streifenförmigen Bilder umfassen oder bestehen aus Informationen und Bilddaten wie Farbe, Helligkeit, etc., die in den als Pixelstreifen ausgestalteten Abbildungsbereichen von geeigneten Elementen erfasst werden.

Erfindungsgemäss ist der zweite Abbildungsbereich als Pixelstreifen ausgebildet, wobei die Kameravorrichtung eine einzige Kamera mit einem Matrixsensor aufweist und alle Abbildungsbereiche auf dem Matrixsensor liegen.

Eine solche Ausgestaltung kann besonders einfach umzusetzen sein und besonders wenig Platz beanspruchen.

Die erfindungsgemäße Lösung kann mit den folgenden, jeweils für sich vorteilhaften und beliebig miteinander kombinierbaren Ausgestaltungen und Weiterentwicklungen weiter verbessert werden.

In einer besonders bevorzugten Variante ist in einem ersten Erfassungszustand ein erster, schräg entgegen der Bewegungsrichtung verlaufender Beobachtungsbereich auf einen ersten, als Pixelstreifen ausgebildeten Abbildungsbereich der Kameravorrichtung abgebildet, wobei in einem zweiten Erfassungszustand ein zweiter Beobachtungsbereich auf einen zweiten Abbildungsbereich der Kameravorrichtung abgebildet ist, wobei in einem dritten Erfassungszustand ein dritter, schräg in der Bewegungsrichtung verlaufender Beobachtungsbereich auf einen dritten, als Pixelstreifen ausgebildeten Abbildungsbereich der Kameravorrichtung abgebildet ist, wobei der zweite Beobachtungsbereich zwischen dem ersten Beobachtungsbereich und dem dritten Beobachtungsbereich liegt. Die Anordnung weist ferner einen Bildprozessor auf, der in den Erfassungszuständen aufgenommene streifenförmige Bilder zu zweidimensionalen Bildern zusammenfügt.

Im ersten und im dritten Erfassungszustand werden entlang des ersten bzw. des dritten Beobachtungsbereichs streifenförmige Bilder aufgenommen, die anschließend zu einem zweidimensionalen Bild zusammengesetzt werden können. Dadurch ist es möglich, nicht nur wie üblich die im zweiten Erfassungszustand aufgenommenen Bilder eines bearbeiteten Bereiches auszuwerten, sondern auch bereits bearbeitete oder unbearbeitete und evtl. schon inspizierte, insbesondere angrenzende Bereiche zu inspizieren.

In einer anderen Ausgestaltung können der erste und der dritte Abbildungsbereich der Kamera jeweils zweidimensional sein, sodass eine darauffolgende Bildbearbeitung einfacher sein kann als bei streifenförmigen Bildern.

Der zweite Abbildungsbereich kann insbesondere senkrecht zur Bewegungsrichtung sein.

In anderen Ausgestaltungen kann der zweite Abbildungsbereich auch schräg in oder entgegen der Bewegungsrichtung verlaufen.

Bei den plattenförmigen Objekten kann es sich insbesondere um Platten und/oder quaderförmige Objekte handeln.

In einer vorteilhaften Ausgestaltung weist die Kameravorrichtung eine einzige Kamera mit einem Matrixsensor auf, wobei alle drei Abbildungsbereiche auf dem Matrixsensor liegen. Bei einer solchen Ausgestaltung kann die Bildverarbeitung besonders einfach sein. Auch der Aufbau kann besonders einfach sein. Ein Matrixsensor kann insbesondere eine Vielzahl von Zeilen und eine Vielzahl von Spalten aufweisen. Alle Bilder können dann mit einem einzigen Matrixsensor aufgenommen werden.

Vorteilhafterweise können die drei Abbildungsbereiche voneinander beabstandet sein.

In einer anderen Ausgestaltung können die drei Abbildungsbereiche zumindest teilweise übereinander liegen.

Als streifenförmige Bilder können Bilder angesehen werden, die eine im Vergleich zu einer Länge eine geringe Breite aufweisen, beispielsweise eine Breite, die um den Faktor 10 geringer ist als die Länge. Ähnliches gilt für Pixelstreifen.

Gemäß der Erfindung weisen die Pixelstreifen eine Breite von einem Pixel auf. Dadurch kann das Zusammensetzen des zweidimensionalen Bildes besonders einfach sein. In einem Beispiel außerhalb der beanspruchten Erfindung kann eine Zeilenkamera verwendet werden.

In einem Beispiel außerhalb der beanspruchten Erfindung können die Pixelstreifen eine Breite von mehreren Pixel aufweisen, beispielsweise zwei bis fünf Pixel. Beim Zusammensetzen des Bildes können die streifenförmigen Bilder in einer ersten Ausführungsform einfach nebeneinander gesetzt werden. In der zweiten Ausführungsform können sich die einzelnen streifenförmigen Bilder auch überlappen, sodass beispielsweise Bilder mit einer höheren Qualität erzeugt werden können.

Der Matrixsensor kann ein CMOS-Sensor sein. Ein solcher kann einfach eingestellt werden. Insbesondere ist es möglich, dass nur einzelne Zeilen angesteuert und/oder ausgelesen werden. Auch die Verwendung von weiteren Sensoren, bei denen einzelne Zeilen oder Bereiche angesteuert und/oder ausgelesen werden können, ist vorteilhaft. Im Vergleich zu anderen Sensoren, bei denen für jedes Bild die gesamte Matrix verwendet wird, ist dadurch eine schnellere Verarbeitung möglich.

Um eine Verstellung einer Optik unnötig zu machen, kann die Anordnung mindestens eine Blende für die Kameravorrichtung umfassen, die eine Schärfentiefe aufweist, die sich mindestens über einen senkrecht zur Bewegungsrichtung gemessenen Inspektionsbereich -nachfolgend als gemessene Breite bezeichnet - des plattenförmigen Objekts erstreckt. Da eine Erhöhung der Schärfentiefe meist mit einer Verringerung der Größe der Apertur erreicht wird und damit die Menge an Licht ebenfalls verringert wird, kann die Anordnung eine Beleuchtungsvorrichtung zur Beleuchtung beispielsweise der Vorderseite und/oder der Rückseite umfassen. Die Beleuchtungsvorrichtung erhöht die Menge an Licht, sodass bei einer großen Schärfentiefe immer noch genügend Licht vorhanden ist.

Die Anordnung kann ein plattenförmiges Objekt mit einer der Kameravorrichtung zugewandten Seitenfläche, einer in die Bewegungsrichtung weisenden Vorderseite und einer entgegen der Bewegungsrichtung weisenden Rückseite umfassen, wobei der Bildprozessor die in den Erfassungszuständen aufgenommenen streifenförmigen Bilder zu zweidimensionalen Bildern der Vorderseite, Seitenfläche bzw. Rückseite zusammenfügt.

Die Kameravorrichtung kann ein Auslesemodul umfassen, das die als Pixelstreifen ausgebildeten Bereiche des Sensors ausliest.

Ferner kann ein Analysemodul vorhanden sein, das die Vorderseite, Rückseite und/oder Seitenfläche des plattenförmigen Objekts bezüglich Defekte prüft.

In einer vorteilhaften Ausgestaltung kann die Anordnung eine Transportvorrichtung zum Transportieren der plattenförmigen Objekte umfassen. Die Transportvorrichtung ist mit der Kameravorrichtung synchronisiert, sodass Bilder im ersten, zweiten bzw. dritten Beobachtungsbereich immer dann aufgenommen werden, wenn sich entsprechende Teile des plattenförmigen Objektes in diesem Beobachtungsbereich befinden.

Zusätzlich oder alternativ kann auch eine Vorrichtung zur Bewegung der Kamera vorhanden sein. In diesem Fall wird die Kamera gegenüber dem Objekt bewegt. Dabei können die plattenförmigen Objekte stationär sein oder sich mit einer anderen Geschwindigkeit als die Kameravorrichtung bewegen.

Im Folgenden wird die Erfindung anhand vorteilhafter Ausgestaltungen mit Bezug auf die Zeichnungen beispielhaft näher erläutert. Die dabei dargestellten vorteilhaften Weiterentwicklungen und Ausgestaltungen sind jeweils voneinander unabhängig und können beliebig miteinander kombiniert werden, je nachdem, wie dies im Anwendungsfall notwendig ist.

Es zeigen:
- Fig. 1: schematische Perspektivansicht zweier plattenförmiger Objekte nach einem Bearbeitungsschritt;
- Fig. 2A - 2E: schematische Ansichten der Anordnung während verschiedener Zeiten in einem ersten Erfassungszustand bzw. Messintervall;
- Fig. 3: eine schematische Ansichten während eines zweiten Erfassungszustands bzw. Messintervalls;
- Fig. 4A - 4E: schematische Ansicht während verschiedener Zeiten in einem dritten Erfassungszustand bzw. Messintervall;
- Fig. 5: eine schematische Ansicht einer Kamera der Anordnung im Detail;
- Fig. 6: eine schematische Ansicht einer weiteren Ausführungsform eines erfindungsgemäßen Verfahrens in einem ersten Erfassungszustand bzw. Messintervall.

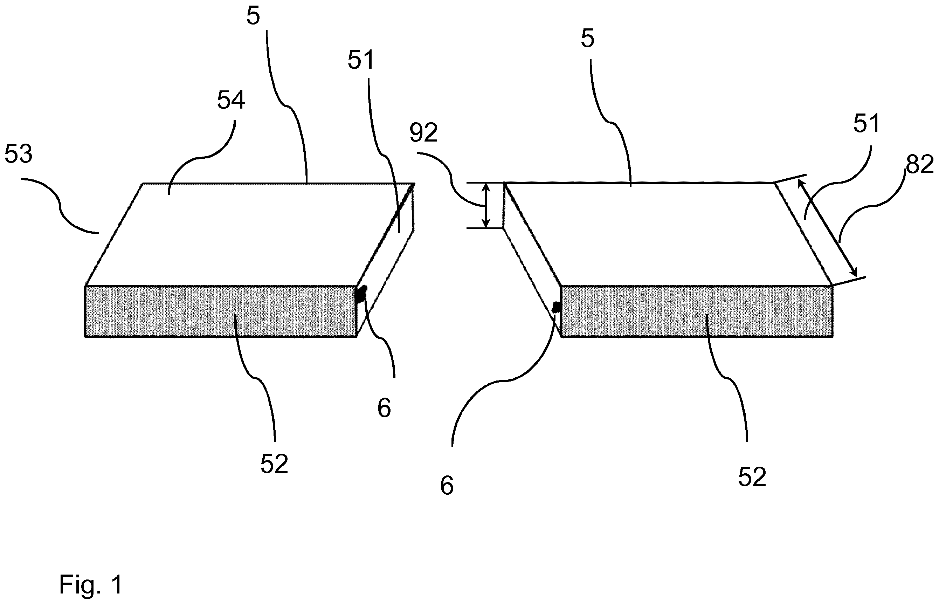

In Fig. 1 sind schematisch zu inspizierende plattenförmige Objekte 5 dargestellt. Sie haben die Form eines Flachkörpers mit einer als Flachseite ausgebildeten Oberseite 54. Daran grenzen eine Vorderseite 51, eine Seitenfläche 52 und eine Rückseite 53 an, die jeweils als Schmalseite ausgebildet sind. Die Seitenfläche 52 wurde bearbeitet. Durch die Bearbeitung sind an der Vorderseite 51 bzw. der Rückseite 53 Defekte 6 entstanden. Bei herkömmlichen Inspektionsvorrichtungen wird lediglich die Seitenfläche 52 nach der Bearbeitung inspiziert. Dabei werden jedoch die Defekte 6 nicht erkannt. Die plattenförmigen Objekte 5 weisen eine entlang einer Höhenrichtung gemessene Höhe 92 und eine senkrecht dazu entlang einer Breitenrichtung gemessene Breite 82 auf. Ein Inspektionsbereich kann die komplette Breite 82 des plattenförmigen Objektes 5 umfassen oder aber auch eine geringere Breite aufweisen.

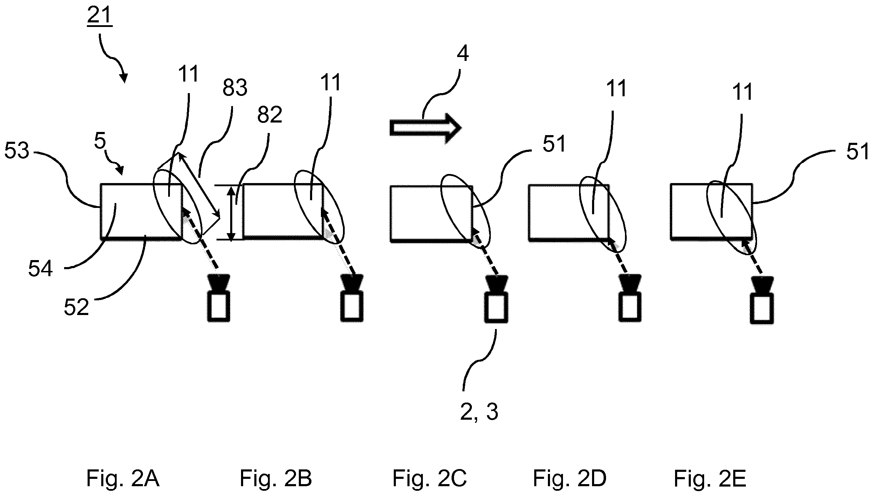

In den Figuren 2A bis 4E sind drei Erfassungszustände 21, 22, 23 dargestellt, bei denen die Vorderseite 51, die Seitenfläche 52 und die Rückseite 53 inspiziert werden. Ein plattenförmiges Objekt 5 bewegt sich dabei entlang einer Bewegungsrichtung 4 relativ zu der Kameravorrichtung 2, die insbesondere eine Kamera 3 umfasst.

In den Figuren 2A bis 2E ist ein erster Erfassungszustand 21 zu verschiedenen Zeitpunkten dargestellt. Während eines ersten Erfassungszustandes 21 werden zu den verschiedenen Zeitpunkten streifenförmige Bilder entlang eines ersten Beobachtungsbereichs 11, der schräg entgegen der Bewegungsrichtung 4 verläuft, aufgenommen. Es werden die einzelnen streifenförmigen Bilder, die von einem Auslesemodul der Kamera 2 ausgelesen wurden, in einem Speicher zwischengespeichert und anschließend aus den streifenförmigen Bildern ein zweidimensionales Bild durch Zusammenfügen der streifenförmigen Bilder erzeugt. Das zweidimensionale Bild, das in diesem Fall die Vorderseite 51 entspricht, kann anschließend in einem Analysemodul bezüglich vorhandener Defekte 6 geprüft werden. Falls Defekte 6 gefunden werden, können entsprechende Maßnahmen eingeleitet werden, beispielsweise kann das plattenförmige Objekt 5 zur Nachbearbeitung aus der Produktionslinie ausgeschleust werden.

Zur Aufnahme der streifenförmigen Bilder des ersten Beobachtungsbereiches 11 wird dieser auf einen als Pixelstreifen ausgebildeten ersten Abbildungsbereich eines Sensors der Kamera 3 abgebildet. Während des gesamten ersten Erfassungszustandes 21, also zu allen in den Figuren 2A bis 2E gezeigten Zeitpunkten, bleibt der erste Abbildungsbereich gleich und wandert nicht. Anstelle eines Matrixsensors könnte also auch ein Zeilensensor benutzt werden.

Um dabei jeweils ein scharfes Bild zu erhalten, weist die Kamera 3 eine Schärfentiefe auf, die sich über mehr als die entlang der senkrecht zur Bewegungsrichtung 4 verlaufenden Breitenrichtung gemessene Breite 82, die einem Inspektionsbereich des plattenförmigen Objekts 5 entsprechen kann, erstreckt. Wie vorstehend erwähnt, kann der Inspektionsbereich die komplette Breite 82 des plattenförmigen Objektes 5 umfassen oder aber auch eine geringere Breite aufweisen. Die Schärfentiefe entspricht in der gezeigten Ausführung etwa der Länge des ersten Beobachtungsbereichs 11. Aufgrund des schrägen Verlaufs des ersten Beobachtungsbereichs 11, wird dieser entlang der Bewegungsrichtung 4 auf die Breitenrichtung projiziert, um zu beurteilen, ob die Schärfentiefe ausreicht.

Eine große Schärfentiefe kann durch eine entsprechend eng dimensionierte Blende erzielt werden. Damit bei einer engen Blende immer noch ausreichend Licht an der Kamera 3 vorhanden ist, kann die Anordnung eine Beleuchtungsvorrichtung aufweisen (nicht dargestellt), die permanent oder nach Bedarf das plattenförmige Objekt 5 beleuchtet oder die Beleuchtungsstärke erhöht.

In einer alternativen Ausgestaltungsform kann während des ersten Erfassungszustands 21 auch die Fokuslänge der Kamera verstellt werden, um zu jedem der Zeitpunkte ein scharfes Bild zu haben. Dies kann etwa notwendig sein, wenn eine entsprechende Schärfentiefe nicht erzielt werden kann.

Die Vorrichtung kann zusätzlich eine Transportvorrichtung umfassen (nicht dargestellt), die die plattenförmigen Objekte 5 an einer Kamera 3 vorbeibewegt. In diesem Fall kann die Transportvorrichtung insbesondere mit der Kamera 3 synchronisiert werden, sodass Bilder entlang der Beobachtungsbereiche 11, 12, 13 nur jeweils dann aufgenommen werden, wenn sich die plattenförmigen Objekte 5 in den entsprechenden Positionen befinden. Alternativ hierzu kann sich die Kamera 3 auch zu den plattenförmigen Objekten 5 bewegen.

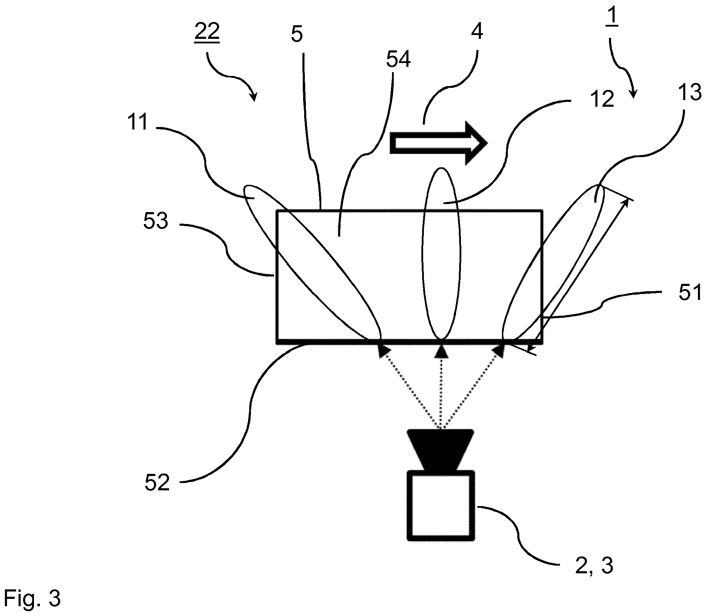

In Fig. 3 ist ein zweiter Erfassungszustand 22 gezeigt, in dem die Seitenfläche 52 inspiziert wird. Dazu misst die Kamera 3 entlang eines zweiten Beobachtungsbereichs 12, der in diesem Fall etwa senkrecht zur Bewegungsrichtung 4 ist. In anderen Ausgestaltungen kann der zweite Beobachtungsbereich 12 auch leicht schräg zur Bewegungsrichtung 4 sein. Der zweite Beobachtungsbereich 12 befindet sich zwischen dem ersten Beobachtungsbereich 11 und einem dritten Beobachtungsbereich 13, der zur Inspektion der Rückseite 53 dient.

In dem in Fig. 3 dargestellten zweiten Erfassungszustand 22 werden in einem zweiten Beobachtungsbereich 12 wieder streifenförmige Bilder aufgenommen. Die einzelnen im zweiten Erfassungszustand 22 aufgenommenen streifenförmigen Bilder werden wieder zu einem zweidimensionalen Bild zusammengefügt, das in diesem Fall die Seitenfläche 52 zeigt. Auch hier kann eine Analyse stattfinden, ob Defekte 6 an der Seitenfläche 52 vorhanden sind.

Alternativ zu der hier gezeigten Ausführungsform, können in dem ersten und dritten Erfassungszustand 21 und 23 jeweils ein zweidimensionales Bild aufgenommen werden. Falls ein einziges zweidimensionales Bild nicht ausreichen sollte, können mehrere aufgenommene zweidimensionale Bilder zusammengefügt werden, um ein Gesamtbild der Seitenfläche 52 zu erhalten.

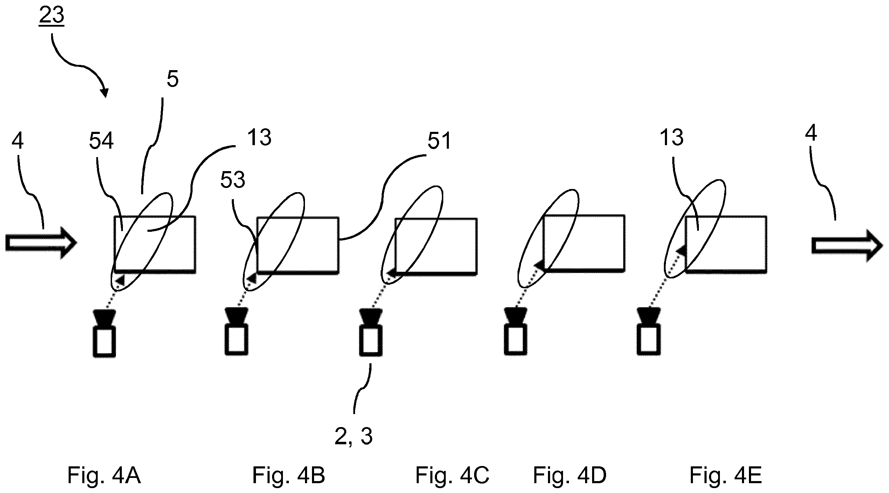

In den Figuren 4A bis 4E ist die Anordnung 1 in einem dritten Erfassungszustand 23 bzw. einem dritten Beobachtungsbereich 13 gezeigt, in dem zu verschiedenen Zeitpunkten streifenförmige Bilder eines dritten Beobachtungsbereichs 13 aufgenommen werden, die später wieder zu einem zweidimensionalen Bild 89 zusammengefügt werden. Der dritte Beobachtungsbereich 13 verläuft schräg in der Bewegungsrichtung 4.

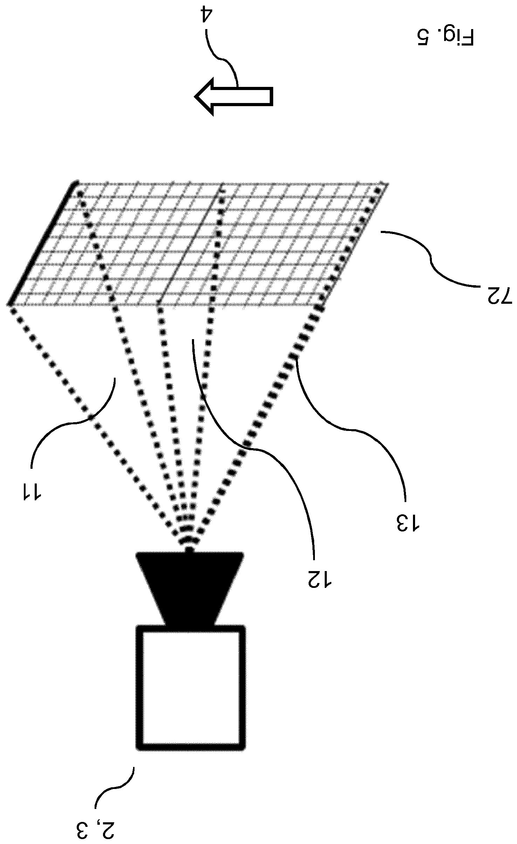

In Fig. 5 ist die Kameravorrichtung 2 bzw. die Kamera 3 mit drei Beobachtungsbereichen 11, 12, 13 schematisch dargestellt.

Bei dem dargestellten ersten Beobachtungsbereich 11 handelt sich um ein flaches Gebiet, dessen Querschnitt sich mit zunehmender Entfernung von der Kamera 3 vergrößert. Es ist etwa fächerförmig. Der erste Beobachtungsbereich 11 verläuft entlang einer ersten Messrichtung, der zweite Beobachtungsbereich 12 erstreckt sich entlang einer zweiten Messrichtung und der dritte Beobachtungsbereich erstreckt sich entlang einer dritten Messrichtung. Die Beobachtungsbereiche 11, 12, 13 bzw. die Messrichtungen schließen jeweils einen Winkel mit der Bewegungsrichtung 4 ein, wobei die erste Messrichtung entgegen der Bewegungsrichtung 4 und schräg zu dieser verläuft und die dritte Messrichtung entlang der Bewegungsrichtung 4 und schräg zu dieser verläuft.

Ferner ist zu erkennen, dass die Kamera 3 insgesamt ein Array 72 erfasst. Die Kamera 3 umfasst einen Matrixsensor mit einer Vielzahl von Bildpunkten bzw. Pixeln, die in Zeilen und dazu senkrechten Spalten angeordnet sind. Der erste Beobachtungsbereich 11 wird auf einen ersten Abbildungsbereich abgebildet, der als Pixelstreifen ausgestaltet ist. Die Breite des Pixelstreifens beträgt dabei erfindungsgemäß einen einzigen Pixel. In diesem Fall wird der Pixelstreifen also von einer einzigen Zeile des Matrixsensors gebildet.

Gemäß einem Beispiel außerhalb der beanspruchten Erfindung kann der Pixelstreifen auch breiter sein und beispielsweise mehrere Pixel, insbesondere 2 bis 5 Pixel breit sein. Bei der in Fig. 11 dargestellten Bildverarbeitung können dann entweder diese breiteren Pixelstreifen direkt nebeneinander gelegt werden oder zur Verbesserung der Qualität überlappende Pixelstreifen benutzt werden.

Alle drei Abbildungsbereiche liegen auf dem Matrixsensor und sind jeweils voneinander beabstandet.

Vorteilhafterweise ist der Matrixsensor als CMOS-Sensor ausgestaltet. Diese Art von Sensor erlaubt eine Ansteuerung einzelner Zeilen, wodurch die Aufnahmegeschwindigkeit erhöht werden kann.

Gemäß einem Beispiel außerhalb der vorliegenden Erfindung kann zur Aufnahme der Bilder in den Abbildungsbereichen auch jeweils eine Zeilenkamera benutzt werden.

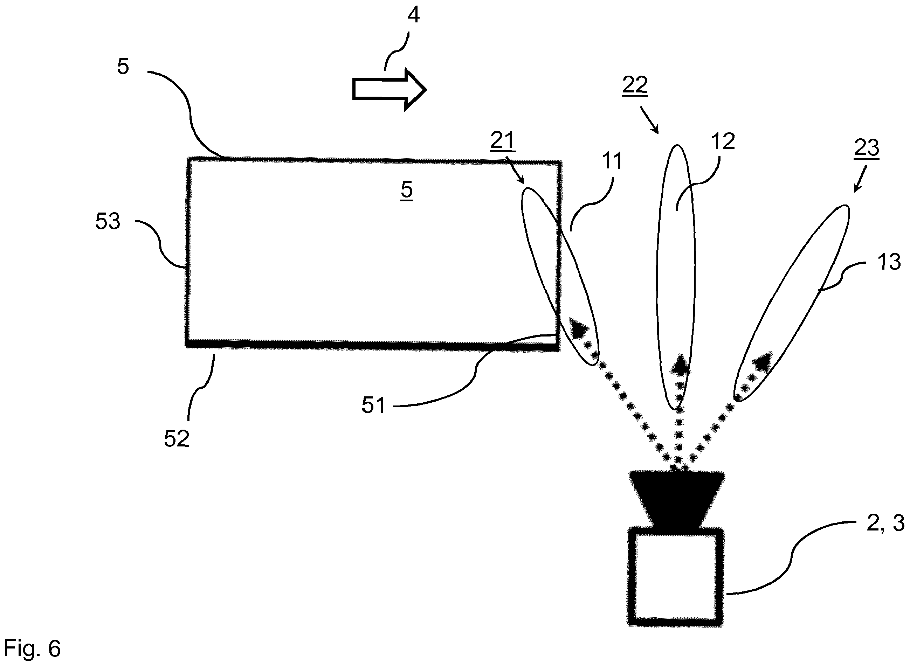

In der Figur 6 ist eine zweite Ausführungsform gezeigt, die leicht von der ersten abweicht. Bei dieser Ausführungsform werden zu allen Zeitpunkten Bilder in allen drei Beobachtungsbereichen 11, 12, 13 aufgenommen. In einem darauffolgenden Schritt können Bilder, die keine wesentlichen Informationen enthalten, verworfen werden.

## Patentansprüche

1. Anordnung zur Inspektion von relativ zu einer Kameravorrichtung (2) in einer Bewegungsrichtung (4) durch wenigstens zwei Beobachtungsbereiche (11, 12) hindurch bewegten plattenförmigen Objekten (5),
wobei die Anordnung die Kameravorrichtung (2) umfasst,
wobei die Kameravorrichtung (2) eine einzige Kamera (3) mit einem Matrixsensor aufweist,
wobei in einem ersten Erfassungszustand (21) zu verschiedenen Zeitpunkten ein schräg entgegen der Bewegungsrichtung (4) verlaufender erster Beobachtungsbereich (11) auf einen, als Pixelstreifen ausgebildeten ersten Abbildungsbereich der Kameravorrichtung (2) zur Aufnahme von streifenförmigen Bildern abgebildet ist,
wobei in einem weiteren Erfassungszustand (22) zu verschiedenen Zeitpunkten ein weiterer Beobachtungsbereich (12) auf einen, als Pixelstreifen ausgebildeten weiteren Abbildungsbereich der Kameravorrichtung (2) zur Aufnahme von streifenförmigen Bildern abgebildet ist,
wobei die Pixelstreifen eine Breite von einem Pixel aufweisen,
wobei die Anordnung ferner einen Bildprozessor aufweist, der die in den Erfassungszuständen (21, 22) aufgenommene streifenförmige Bilder zu zweidimensionalen Bildern zusammenfügt und
wobei der erste und der weitere Abbildungsbereiche auf dem Matrixsensor liegen.

2. Anordnung nach Anspruch 1, **dadurch gekennzeichnet, dass** in einem ersten Erfassungszustand (21) ein erster, schräg entgegen der Bewegungsrichtung (4) verlaufender Beobachtungsbereich (11) auf einen ersten, als Pixelstreifen ausgebildeten Abbildungsbereich der Kameravorrichtung (2) abgebildet ist, wobei in einem zweiten Erfassungszustand (22) ein zweiter Beobachtungsbereich (12) auf einen zweiten Abbildungsbereich der Kameravorrichtung (2) abgebildet ist, wobei in einem dritten Erfassungszustand (23) ein dritter, schräg in der Bewegungsrichtung (4) verlaufender Beobachtungsbereich (13) auf einen dritten, als Pixelstreifen ausgebildeten Abbildungsbereich der Kameravorrichtung (2) abgebildet ist, wobei der zweite Beobachtungsbereich (12) zwischen dem ersten Beobachtungsbereich (11) und dem dritten Beobachtungsbereich (13) liegt, und wobei die Anordnung ferner einen Bildprozessor aufweist, der in den Erfassungszuständen (21, 22, 23) aufgenommene streifenförmige Bilder zu zweidimensionalen Bildern zusammenfügt.

3. Anordnung nach Anspruch 2, **dadurch gekennzeichnet, dass** die drei Abbildungsbereiche voneinander beabstandet sind.

4. Anordnung nach einem der Ansprüche 1 bis 3, **dadurch gekennzeichnet, dass** die Anordnung mindestens eine Blende für die Kameravorrichtung (2) umfasst, die eine Schärfentiefe aufweist, die sich mindestens über die senkrecht zur Bewegungsrichtung (4) gemessene Breite (82) des plattenförmigen Objekts (5) erstreckt, und eine Beleuchtungsvorrichtung zur Beleuchtung der Vorderseite (51) und/oder der Rückseite (53).

5. Anordnung nach einem der Ansprüche 1 bis 4, **dadurch gekennzeichnet, dass** bei dem plattenförmiges Objekt eine Seitenfläche (52) der Kameravorrichtung (2) zugewandt ist, eine Vorderseite (51) in die Bewegungsrichtung (4) weist und eine Rückseite (53) entgegen der Bewegungsrichtung (4) weist und der Bildprozessor die in den Erfassungszuständen (21, 22, 23) aufgenommenen streifenförmigen Bilder zu zweidimensionalen Bildern der Vorderseite (51), Seitenfläche (52) bzw. Rückseite (53) zusammenfügt.

6. Anordnung nach einem der Ansprüche 1 bis 5, **dadurch gekennzeichnet, dass** die Anordnung eine Transportvorrichtung zum Transportieren der plattenförmigen Objekte (5) umfasst, mit der die Kameravorrichtung (2) synchronisiert ist.

7. Verfahren zur Inspektion von relativ zu einer Kameravorrichtung (2) in einer Bewegungsrichtung (4) durch wenigstens zwei Beobachtungsbereiche (11, 12) hindurch bewegten plattenförmigen Objekten (5), wobei die Kameravorrichtung (2) eine einzige Kamera (3) mit einem Matrixsensor aufweist, wobei in einem ersten Erfassungszustand (21) entlang eines, schräg entgegen der Bewegungsrichtung (4) verlaufenden ersten Beobachtungsbereichs (11) zu verschiedenen Zeitpunkten Pixelstreifen auf dem Matrixsensor zum Aufnehmen von streifenförmigen Bilder aufgenommen werden und wobei in einem weiteren Erfassungszustand (22) entlang eines weiteren Beobachtungsbereichs (12) zu verschiedenen Zeitpunkten Pixelstreifen auf dem Matrixsensor zum Aufnehmen von streifenförmige Bilder aufgenommen werden, wobei die Pixelstreifen jeweils eine Breite von einem Pixel aufweisen und von einem Bildprozessor jeweils die in den einzelnen Erfassungszuständen (21, 22) aufgenommenen streifenförmigen Bilder zu einem zweidimensionalen Bild zusammengesetzt werden.

8. Verfahren zur Inspektion nach Anspruch 7
**dadurch gekennzeichnet, dass** in einem
ersten Erfassungszustand (21) entlang eines ersten, schräg entgegen der Bewegungsrichtung (4) verlaufenden Beobachtungsbereichs (11) streifenförmige Bilder aufgenommen werden, wobei in einem zweiten Erfassungszustand (22) entlang eines zweiten Beobachtungsbereichs (12) weitere streifenförmige Bilder aufgenommen werden, wobei in einem dritten Erfassungszustand (23) entlang eines dritten, schräg in der Bewegungsrichtung (4) verlaufenden Beobachtungsbereichs (13) weitere streifenförmige Bilder aufgenommen werden, wobei der Beobachtungsbereich des zweite Erfassungszustandes (22) zwischen dem Beobachtungsbereich (11) des ersten Erfassungszustandes (21) und dem Beobachtungsbereich (13) des dritten Erfassungszustandes (23) liegt, und wobei jeweils die in den einzelnen Erfassungszuständen (21, 22, 23) aufgenommenen streifenförmigen Bilder zu einem zweidimensionalen Bild zusammengesetzt werden.

## Claims

1. Arrangement for inspecting plate-shaped objects (5) moved relative to a camera device in a movement direction (4) through at least two observation areas (11, 12),
wherein the arrangement comprises the camera device (2),
wherein the camera device (2) comprises a single camera (3) with a matrix sensor, wherein, in a first detection state (21), a first observation area (11) which extends at an oblique angle counter to the movement direction (4) is displayed on a first image area of the camera device (2), which area is formed by pixel strips, at different points in time in order to record strip-shaped images,
wherein, in a further detection state (22), a further observation area (12) is displayed on a further image area of the camera device (2), which area is formed by pixel strips, at different points in time in order to record strip-shaped images,
wherein the pixel strips have a width of one pixel,
wherein the arrangement also comprises an image processor which joins the strip-shaped images recorded in the detection states (21, 22) to form two-dimensional images and
wherein the first and the further image areas are located on the matrix sensor.

2. Arrangement according to claim 1, **characterized in that,** in a first detection state (21), a first observation area (11) which extends at an oblique angle counter to the movement direction (4) is displayed on a first image area of the camera device (2), which area is in the form of pixel strips, wherein, in a second detection state (22), a second observation area (12) is displayed on a second image area of the camera device (2), wherein, in a third detection state (23), a third observation area (13) which extends at an oblique angle in the movement direction (4) is displayed on a third image area of the camera device (2), which image area is in the form of pixel strips, wherein the second observation area (12) is located between the first observation area (11) and the third observation area (13), and wherein the arrangement also has an image processor which joins the strip-shaped images recorded in the detection states (21, 22, 23) to form two-dimensional images.

3. Arrangement according to claim 2, **characterized in that** the three image areas are spaced apart.

4. Arrangement according to any of claims 1 to 3, **characterized in that** the arrangement comprises at least one aperture for the camera device (2) which has a depth of field extending at least over the width (82) of the plate-shaped object (5) measured perpendicularly to the movement direction (4), and an illumination device for illuminating the front side (51) and/or the rear side (53).

5. Arrangement according to any of claims 1 to 4, **characterized in that,** in the case of the plate-shaped object, a side surface (52) faces the camera device (2), a front side (51) faces the movement direction (4), and a rear side (53) faces away from the movement direction (4), and the image processor joins the strip-shaped images recorded in the detection states (21, 22, 23) to form two-dimensional images of the front side (51), the side surface (52), and the rear side (53).

6. Arrangement according to any of claims 1 to 5, **characterized in that** the arrangement comprises a transportation device for transporting the plate-shaped objects (5) which is synchronized with the camera device (2).

7. Method for inspecting plate-shaped objects (5) moved relative to a camera device (2) in a movement direction (4) through at least two observation areas (11, 12), wherein the camera device (2) comprises a single camera (3) with a matrix sensor, wherein, in a first detection state (21), pixel strips along a first observation area (11) which extends at an oblique angle to the movement direction (4) are recorded at different points in time on the matrix sensor in order to record strip-shaped images and wherein, in a further detection state (22), pixel strips along a further observation area (12) are recorded at different points in time on the matrix sensor in order to record strip-shaped images, wherein the pixel strips each have a width of one pixel and the strip-shaped images recorded in the respective individual detection states (21, 22) are joined by an image processor to form a two-dimensional image.

8. Method for inspecting according to claim 7, **characterized in that,** in a first detection state (21), strip-shaped images are recorded along a first observation area (11) which extends at an oblique angle counter to the movement direction (4), wherein, in a second detection state (22), further strip-shaped images are recorded along a second observation area (12), wherein, in a third detection state (23), further strip-shaped images are recorded along a third observation area (13) which extends at an oblique angle in the movement direction (4), wherein the observation area of the second detection state (22) is located between the observation area (11) of the first detection state (21) and the observation area (13) of the third detection state (23), and wherein the strip-shaped images recorded in the respective individual detection states (21, 22, 23) are joined to form a two-dimensional image.

## Revendications

1. Agencement pour l'inspection d'objets en forme de plaque (5) déplacés par rapport à un dispositif de caméra (2) dans une direction de déplacement (4) à travers au moins deux zones d'observation (11, 12),
l'agencement comprenant le dispositif de caméra (2),
le dispositif de caméra (2) présentant une seule caméra (3) dotée d'un capteur matriciel,
dans un premier état de détection (21), à différents moments, une première zone d'observation (11) s'étendant obliquement à l'opposé de la direction de déplacement (4) étant reproduite sur une première zone de représentation, se présentant sous la forme de bandes de pixels, du dispositif de caméra (2) pour l'enregistrement d'images en forme de bandes,
dans un autre état de détection (22), à différents moments, une autre zone d'observation (12) étant reproduite sur une autre zone de représentation, se présentant sous la forme de bandes de pixels, du dispositif de caméra (2) pour l'enregistrement d'images en forme de bandes,
les bandes de pixels présentant une largeur d'un pixel,
l'agencement présentant en outre un processeur d'image qui assemble les images en forme de bandes enregistrées dans les états de détection (21, 22) en images bidimensionnelles et
la première et l'autre zone de représentation étant situées sur le capteur matriciel.

2. Agencement selon la réalisation 1, **caractérisé en ce que,** dans un premier état de détection (21), une première zone d'observation (11) s'étendant obliquement à l'opposé de la direction de déplacement (4) est reproduite sur une première zone de représentation, se présentant sous la forme de bandes de pixels, du dispositif de caméra (2), dans un deuxième état de détection (22), une deuxième zone d'observation (12) étant reproduite sur une deuxième zone de représentation du dispositif de caméra (2), dans un troisième état de détection (23), une troisième zone d'observation (13) s'étendant obliquement dans la direction de déplacement (4) étant reproduite sur une troisième zone de représentation, se présentant sous la forme de bandes de pixels, du dispositif de caméra (2), la deuxième zone d'observation (12) étant située entre la première zone d'observation (11) et la troisième zone d'observation (13), et l'agencement présentant en outre un processeur d'images qui assemble des images en forme de bandes enregistrées dans les états de détection (21, 22, 23) en images bidimensionnelles.

3. Agencement selon la revendication 2, **caractérisé en ce que** les trois zones de représentation sont disposées à distance les unes des autres.

4. Agencement selon l'une des revendications 1 à 3, **caractérisé en ce que** l'agencement comprend au moins un diaphragme pour le dispositif de caméra (2), qui présente une profondeur de champ qui s'étend au moins sur la largeur (82) de l'objet en forme de plaque (5), mesurée perpendiculairement à la direction de déplacement (4), et un dispositif d'éclairage pour éclairer la face avant (51) et/ou la face arrière (53).

5. Agencement selon l'une des revendications 1 à 4, **caractérisé en ce que,** dans le cas de l'objet en forme de plaque, une surface latérale (52) est tournée vers le dispositif de caméra (2), une face avant (51) est orientée dans la direction de déplacement (4) et une face arrière (53) est orientée à l'opposé de la direction de déplacement (4) et le processeur d'images assemble les images en forme de bandes enregistrées dans les états de détection (21, 22, 23) en images bidimensionnelles de la face avant (51), de la surface latérale (52) ou de la face arrière (53).

6. Agencement selon l'une des revendications 1 à 5, **caractérisé en ce que** l'agencement comprend un dispositif de transport pour transporter les objets en forme de plaque (5) avec lesquels est synchronisé le dispositif de caméra (2).

7. Procédé d'inspection d'objets en forme de plaque (5) déplacés par rapport à un dispositif de caméra (2) dans une direction de déplacement (4) à travers au moins deux zones d'observation (11, 12), le dispositif de caméra (2) présentant une seule caméra (3) dotée d'un capteur matriciel, dans un premier état de détection (21), le long d'une première zone d'observation (11) s'étendant obliquement à l'opposé de la direction de déplacement (4), des bandes de pixels étant enregistrées à différents moments sur le capteur matriciel pour enregistrer des images en forme de bandes et, dans un autre état de détection (22), des bandes de pixels étant enregistrées à différents moments sur le capteur matriciel pour enregistrer des images en forme de bandes le long d'une autre zone d'observation (12), les bandes de pixels présentant chacune une largeur d'un pixel et les images en forme de bandes enregistrées dans les différents états de détection (21, 22) étant respectivement assemblées en une image bidimensionnelle par un processeur d'images.

8. Procédé d'inspection selon la revendication 7, **caractérisé en ce que,** dans un premier état de détection (21), des images en forme de bandes sont enregistrées le long d'une première zone d'observation (11) s'étendant obliquement à l'opposé de la direction de déplacement (4), dans un deuxième état de détection (22), d'autres images en forme de bandes étant enregistrées le long d'une deuxième zone d'observation (12), dans un troisième état de détection (23), d'autres images en forme de bandes étant enregistrées le long d'une troisième zone d'observation (13) s'étendant obliquement dans la direction de déplacement (4), la zone d'observation du deuxième état de détection (22) étant située entre la zone d'observation (11) du premier état de détection (21) et la zone d'observation (13) du troisième état de détection (23), et les images en forme de bandes enregistrées dans les différents états de détection (21, 22, 23) étant respectivement assemblées en une image bidimensionnelle.
